# EUROPEAN PATENT APPLICATION

(11) **EP 4 310 857 A1**
(43) Date of publication of application: **24.01.2024**
(21) Application number: 22771329.4
(22) Date of filing: 11.03.2022
(51) Int. Cl.: G16H 40/00, G06Q 10/08

(54) **INVENTORY MANAGEMENT SYSTEM, INVENTORY MANAGEMENT DEVICE, INVENTORY MANAGEMENT METHOD, AND INVENTORY MANAGEMENT PROGRAM**

(30) Priority: 17.03.2021 JP 2021043822
(71) Applicant: Nitto Denko Corporation, Ibaraki-shi, Osaka 567-8680 (JP)
(72) Inventor: TSUJI, Takashi, Ibaraki-shi, Osaka 567-8680 (JP)
(74) Representative: Hoffmann Eitle
(86) International application number: PCT/JP2022/011066
(87) International publication number: WO 2022/196581

(57) **Abstract**

An inventory management system has an inventory management device and a terminal device, and the inventory management device includes an inventory status generation unit configured to generate information indicating an inventory status of a medical device for each of medical institutions, by referring to a required inventory storage unit storing information indicating a required inventory quantity of the medical device for each of the medical institutions, and a state management storage unit storing state management information associating a unique ID of the medical device dispatched to one medical institution with a collection status of the medical device paid out from the one medical institution to a patient for each of the medical institutions, and an output unit configured to output the information indicating the inventory status to the terminal device.

## Description

### TECHNICAL FIELD

The present invention relates to inventory management systems, inventory management devices, inventory management methods, and inventory management programs.

### BACKGROUND ART

Conventionally, in medical devices, there are demands to individually identify the devices, to clearly record a process from manufacture until disposal through distribution and sale, and to enable checking of history information by following-back from the device.

### PRIOR ART DOCUMENTS

### PATENT DOCUMENTS

Patent Document 1: Japanese Patent No. 6095900
Patent Document 2: Japanese Patent No. 6490639
Patent Document 3: Japanese Patent No. 678034

### DISCLOSURE OF THE INVENTION

### PROBLEM TO BE SOLVED BY THE INVENTION

In the manufacture, sales, or the like of the medical devices, it is necessary to hold a distributor's inventory according to each business condition. However, because it is difficult to predict the number, timing, or the like of patients who will visit a medical institution, it is impossible to grasp usage demands of the medical devices, delivery timings of the medical devices, or the like. For this reason, considerable cost and labor are conventionally spent on inventory management.

According to one aspect, it is an object to reduce a load of inventory management.

### MEANS OF SOLVING THE PROBLEM

According to one aspect of the present invention, an inventory management system has an inventory management device and a terminal device, and the inventory management device includes an inventory status generation unit configured to generate information indicating an inventory status of a medical device for each of a plurality of medical institutions, by referring to a required inventory storage unit storing information indicating a required inventory quantity of the medical device for each of the plurality of medical institutions, and a state management storage unit storing state management information associating a unique ID of the medical device dispatched to one medical institution with a collection status of the medical device paid out from the one medical institution to a patient for each of the plurality of medical institutions, and an output unit configured to output the information indicating the inventory status to the terminal device.

### EFFECTS OF THE INVENTION

The load of inventory management can be reduced.

### BRIEF DESCRIPTION OF DRAWINGS

[FIG. 1] FIG. 1 is a diagram for explaining an outline of an inventory management system according to a first embodiment.
[FIG. 2] FIG. 2 is a diagram illustrating an example of a system configuration of the inventory management system according to the first embodiment.
[FIG. 3] FIG. 3 is a diagram illustrating an example of a hardware configuration of the inventory management device according to the first embodiment.
[FIG. 4] FIG. 4 is a diagram illustrating an example of a required inventory database according to the first embodiment.
[FIG. 5] FIG. 5 is a diagram illustrating an example of a state management database according to the first embodiment.
[FIG. 6] FIG. 6 is a diagram for explaining functions of an inventory management device according to the first embodiment.
[FIG. 7] FIG. 7 is a sequence diagram for explaining an operation of the inventory management system according to the first embodiment.
[FIG. 8] FIG. 8 is a diagram illustrating an example of an input screen for transmitting a dispatch notification according to the first embodiment.
[FIG. 9] FIG. 9 is a diagram illustrating a display example of a replenishment instruction according to the first embodiment.
[FIG. 10] FIG. 10 is a diagram illustrating an example of a system configuration of the inventory management system according to a second embodiment.
[FIG. 11] FIG. 11 is a diagram illustrating an example of the state management database according to the second embodiment.
[FIG. 12] FIG. 12 is a diagram illustrating an example of an analysis result database according to the second embodiment.
[FIG. 13] FIG. 13 is a diagram for explaining functions of the inventory management device according to the second embodiment.
[FIG. 14] FIG. 14 is a sequence diagram for explaining the operation of the inventory management system according to the second embodiment.
[FIG. 15] FIG. 15 is a diagram illustrating an example of the input screen for transmitting a payout notification according to the second embodiment.
[FIG. 16] FIG. 16 is a diagram illustrating a display example of the replenishment instruction according to the second embodiment.

### MODE OF CARRYING OUT THE INVENTION

### (First Embodiment)

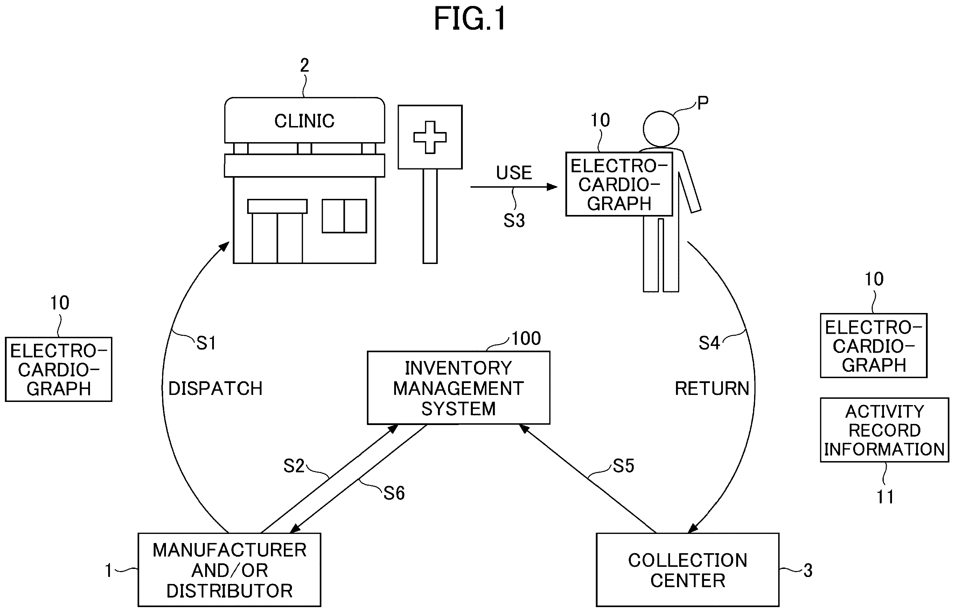

Hereinafter, a first embodiment will be described with reference to the drawings. FIG. 1 is a diagram illustrating an outline of an inventory management system according to a first embodiment.

In the following embodiment, an electrocardiograph 10 will be described as an example of a medical device that is a target of inventory management by an inventory management system 100 according to the present embodiment.

In FIG. 1, the electrocardiograph 10 is manufactured and/or sold by a manufacturer and/or distributor 1. The manufacturer and/or distributor 1 refers to a supplier who distributes to medical institutions, and does not indicate a distinction among a manufacturer, a manufacturer and distributor, and a distributor as defined in the Pharmaceutical and Medical Device Act.

The electrocardiograph 10 according to the present embodiment is a single-use medical device that is collected and discarded after being worn on a body of a patient for a certain period of time.

The electrocardiograph 10 according to the present embodiment includes a storage device that stores measured electrocardiogram waveform data or the like. In addition, identification information for identifying the electrocardiograph 10 is stored in the storage device or the like of the electrocardiograph 10. In the present embodiment, the identification information for uniquely identifying the electrocardiograph 10 is stored in the storage device of all of the electrocardiographs 10 manufactured by the manufacturer and/or distributor 1.

A method of assigning the identification information to the electrocardiograph 10 is not limited to the method described above. The method of assigning the identification information to the electrocardiograph 10 may be any method as long as the electrocardiograph 10 can be associated with the identification information for uniquely identifying the electrocardiograph 10. In the following description, the identification information (unique ID) for uniquely identifying the electrocardiograph 10 may also be referred to as a device ID.

In the present embodiment, the electrocardiograph 10 having the device ID assigned thereto is dispatched from the manufacturer and/or distributor 1 to a medical institution 2 (procedure S1).

In this state, the inventory management system 100 according to the present embodiment receives the device ID of the dispatched electrocardiograph 10 and the identification information for uniquely identifying the medical institution that are input from the manufacturer and/or distributor 1, and holds information associating the device ID and the identification information of the medical institution (procedure S2). In the following description, the identification information for uniquely identifying the medical institution may also be referred to as a medical institution ID.

In the medical institution 2, the electrocardiograph 10 is used on a patient P (procedure S3), and the patient P wears the electrocardiograph 10 to collect the electrocardiogram waveform data from the electrocardiograph 10.

Then, when the measurement of the electrocardiogram waveform for a certain period of time ends, the patient P returns the electrocardiograph 10 to a collection center 3 by post or the like (procedure S4). In this state, the patient P provides the collection center 3 with activity record information 11 indicating the patient's own activity contents during the period of time in which the electrocardiograph 10 is worn by the patient P.

The activity record information 11 is a document or the like, for example, and may be forwarded by post from the patient P to the collection center 3, together with the electrocardiograph 10. In addition, the electrocardiograph 10 may be returned from the patient P who used the electrocardiograph 10 to the medical institution 2, and may be forwarded by post from the medical institution 2 to the collection center 3.

The collection center 3 according to the present embodiment reads the electrocardiogram waveform and the device ID stored in the storage device of the used electrocardiograph 10, and thereafter notifies the device ID of the electrocardiograph 10 to the inventory management system 100 (procedure S5).

Because the device ID of the electrocardiograph 10 and a medical institution ID of the medical institution 2 are associated with each other and held in the inventory management system 100, the inventory management system 100 detects that an inventory of the electrocardiograph 10 in the medical institution 2 decreased by one when notified of the device ID.

Then, the inventory management system 100 instructs the manufacturer and/or distributor 1 to replenish the inventory of the electrocardiograph 10 in the medical institution 2 (procedure S6).

A timing at which the inventory management system 100 instructs the manufacturer and/or distributor 1 to replenish the inventory may be arbitrarily determined between the manufacturer and/or distributor 1 and the medical institution 2. More particularly, a replenish instruction to replenish the inventory may be issued in units of a certain period of time, for example.

As described above, in the inventory management system 100 according to the present embodiment, the device ID for identifying the electrocardiograph 10 is assigned to the single-use electrocardiograph 10, and the device ID and the medical institution ID for identifying the medical institution to which the electrocardiograph 10 is dispatched are managed in association with each other. Hence, according to the present embodiment, a user of the inventory management system 100 can grasp a dispatch destination of the electrocardiograph 10.

Further, when the electrocardiograph 10 is collected and discarded, the inventory management system 100 according to the present embodiment receives a notification indicating that the electrocardiograph 10 has been collected together with the device ID of the electrocardiograph 10, and identifies the medical institution from which the electrocardiograph 10 was collected. Accordingly, in the inventory management system 100 according to the present embodiment, the user of the inventory management system 100 can grasp usage demands of the electrocardiograph 10 in the medical institution.

Moreover, the inventory management system 100 according to the present embodiment can notify a quantity of the electrocardiograph 10 to be replenished in the medical institution, with respect to the manufacturer and/or distributor 1, based on the inventory and the usage demands of the electrocardiograph 10 required in the medical institution.

As described above, according to the present embodiment, neither the manufacturer and/or distributor 1 nor the medical institution 2 needs to count the inventory and the usage demands of the electrocardiograph 10, and it is possible to easily manage the inventory and reduce a load of the inventory management.

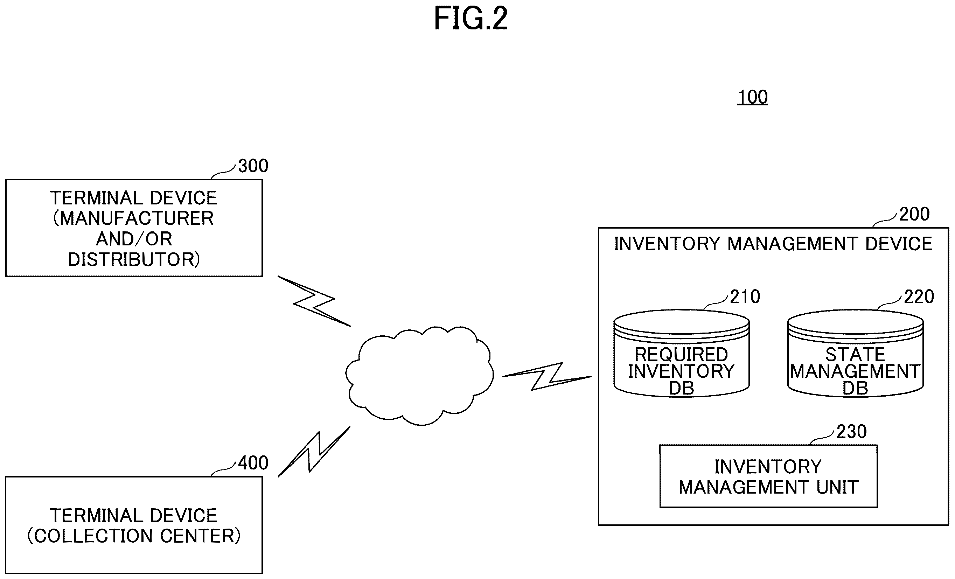

Hereinafter, a system configuration of the inventory management system 100 according to the present embodiment will be described with reference to FIG. 2. FIG. 2 is a diagram illustrating an example of the system configuration of the inventory management system according to the first embodiment.

The inventory management system 100 according to the present embodiment includes an inventory management device 200, a terminal device 300, and a terminal device 400. In the inventory management system 100, the inventory management device 200 is connected to the terminal device 300 and the terminal device 400 via a network, such as the Internet or the like.

In the following description, it is assumed that the terminal device 300 is a terminal device mainly used in the manufacturer and/or distributor 1, and that the terminal device 400 is a terminal device mainly used in the collection center 3. Further, in the following description, it is assumed that the manufacturer of the electrocardiograph 10 is also the distributor thereof, but the manufacturer and the distributor may exist independently.

The inventory management device 200 according to the present embodiment includes a required inventory database 210, a state management database 220, and an inventory management unit 230.

The required inventory database 210 stores required inventory information indicating a required inventory quantity for each medical institution. The required inventory database 210 according to the present embodiment may be stored in advance in the inventory management device 200.

The state management database 220 is provided for each medical institution, and stores state management information including a collection status of the electrocardiograph 10, dispatched from the manufacturer and/or distributor 1 to the medical institution 2, and collected from the patient. In addition, the state management database 220 according to the present embodiment may be generated by inputting the state management information of the electrocardiograph 10 from the terminal device 300.

The inventory management unit 230 receives, from the terminal device 300, a notification of dispatch of the electrocardiograph 10 from the manufacturer and/or distributor 1 to the medical institution 2, and updates the state management database 220. In addition, the inventory management unit 230 receives, from the terminal device 400, a notification of collection of the electrocardiograph 10, and updates the state management database 220.

Further, the inventory management unit 230 computes a used quantity of the electrocardiograph 10, based on the required inventory quantity stored in the required inventory database 210 and the notification from the terminal device 400, generates information indicating an inventory status of the medical institution 2, and transmits the information to the terminal device 300. The information indicating the inventory status includes a current inventory quantity (inventory amount) of the electrocardiograph 10 in the medical institution 2, and a replenishment instruction of the electrocardiograph 10 for setting the current inventory quantity to the required inventory quantity.

Accordingly, the manufacturer and/or distributor 1, who is a user of the terminal device 300, does not need to confirm the used quantity of the electrocardiograph 10 with respect to the medical institution 2, and the load of the inventory management can be reduced. The quantity of the electrocardiograph 10 in the present embodiment indicates the number of the electrocardiographs 10.

In addition, in the example illustrated in FIG. 2, one terminal device 300 and one terminal device 400 are included in the inventory management system 100, but the numbers thereof are not limited to one. An arbitrary number of terminal devices 300 and an arbitrary number of terminal devices 400 may be included in the inventory management system 100.

Moreover, in the example illustrated in FIG. 2, the inventory management device 200 includes the required inventory database 210 and the state management database 220, but the configuration is not limited thereto. A part or all of the required inventory database 210 and the state management database 220 may be provided in an external device other than the inventory management device 200. In this case, the inventory management device 200 and the external device communicate with each other via a network or the like.

Further, the inventory management device 200 according to the present embodiment may be configured using a plurality of information processing apparatuses. In other words, the functions of the inventory management device 200 may be implemented by the plurality of information processing apparatuses.

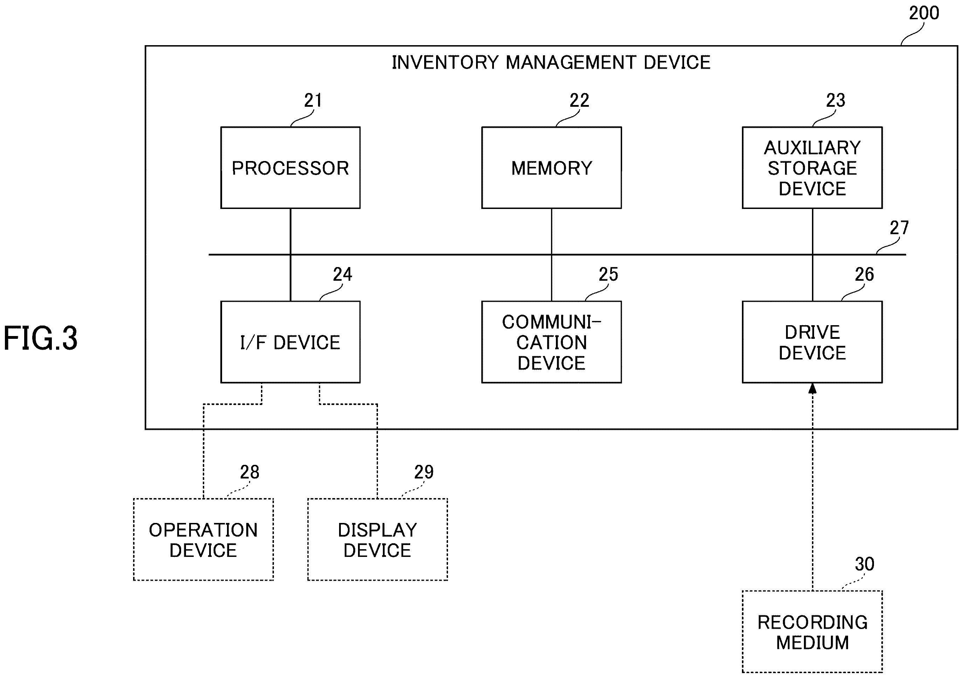

Next, a hardware configuration of the inventory management device 200 according to the present embodiment will be described with reference to FIG. 3. FIG. 3 is a diagram illustrating an example of the hardware configuration of the inventory management device according to the first embodiment.

The inventory management device 200 according to the present embodiment includes a processor 21, a memory 22, an auxiliary storage device 23, an interface (I/F) device 24, a communication device 25, and a drive device 26. These hardware components of the inventory management device 200 are connected to one another via a bus 27.

The processor 21 includes various computing devices, such as a central processing unit (CPU), a graphics processing unit (GPU), or the like. The processor 21 reads various programs (for example, a learning program or the like) into the memory 22, and executes the programs.

The memory 22 includes a main storage device, such as a read only memory (ROM), a random access memory (RAM), or the like. The processor 21 and the memory 22 form a so-called computer, and the inventory management unit 230 is implemented by the computer when the processor 21 executes the various programs read into the memory 22.

The auxiliary storage device 23 stores various programs, and various types of data used when the various programs are executed by the processor 21. For example, the required inventory database 210 and the state management database 220 are implemented in the auxiliary storage device 23.

The I/F device 24 is a coupling device that connects an operation device 28 and a display device 29, which are examples of external devices, to the inventory management device 200. The I/F device 24 receives an operation on the inventory management device 200, via the operation device 28. In addition, the I/F device 24 may output a result of processing performed by the inventory management device 200, and display the result to a manager of the inventory management device 200, via the display device 29.

The communication device 25 is a communication device for communicating with other devices (the terminal devices 300 and 400 in the present embodiment).

The drive device 26 is a device on which a recording medium 30 is set. The recording medium 30 includes a medium for optically, electrically, or magnetically recording information, such as a CD-ROM, a flexible disk, a magneto-optical disk, or the like. Moreover, the recording medium 30 may include a semiconductor memory or the like that electrically records information, such as a ROM, a flash memory, or the like.

The various programs are installed in the auxiliary storage device 23, when a distributed recording medium 30 is set in the drive device 26, and the various programs recorded in the recording medium 30 are read by the drive device 26, for example. Alternatively, the various programs are installed in the auxiliary storage device 23 when downloaded from a network via the communication device 25.

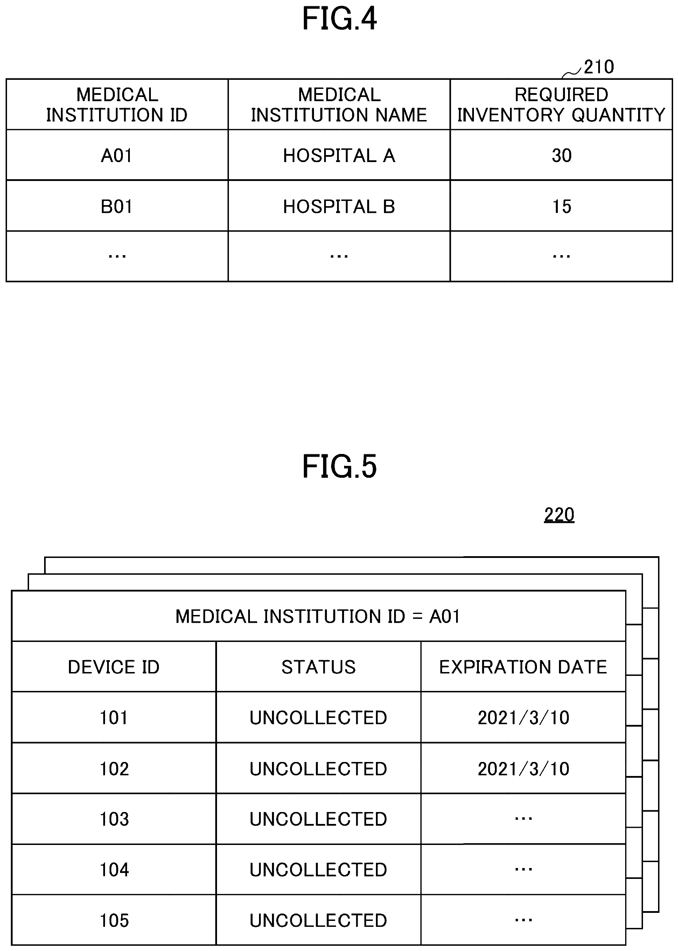

Next, each database of the inventory management device 200 according to the present embodiment will be described with reference to FIG. 4 and FIG. 5.

FIG. 4 is a diagram illustrating an example of the required inventory database according to the first embodiment. The required inventory database 210 according to the present embodiment includes a medical institution ID, a medical institution name, a required inventory quantity, or the like, as information items, and the item "medical institution ID" and each of the other items are associated with each another. In the present embodiment, information including a value of the item "medical institution ID" and values of the other items, is the required inventory information.

The value of the item "medical institution ID" is identification information for identifying the medical institution. The value of the item "medical institution name" indicates a name of the medical institution identified by the medical institution ID. The value of the item "required inventory quantity" indicates an inventory quantity of the electrocardiograph 10 required in the medical institution identified by the medical institution ID. The inventory quantity of the electrocardiograph 10 is set in advance according to a scale of the medical institution, medical departments, or the like, for example. Further, the inventory quantity of the electrocardiograph 10 may be a value that is updated at regular intervals, for example.

In addition, the inventory quantity according to the present embodiment may be stored as a deposit inventory of the medical institution 2, for example. The deposit inventory indicates the electrocardiographs 10 owned by the manufacturer and/or distributor 1 but stocked in the medical institution 2.

In the example illustrated in FIG. 4, the medical institution identified by a medical institution ID "A01" is "hospital A", and the required inventory quantity is 30.

FIG. 5 is a diagram illustrating an example of the state management database according to the first embodiment. The state management database 220 according to the present embodiment is provided for each medical institution, and FIG. 5 illustrates the state management database 220 of the medical institution identified by the medical institution ID "A01".

The state management database 220 according to the present embodiment includes a medical institution ID, a device ID, a status, and an expiration date, as the information items. The item "medical institution ID" is associated with the item "device ID", and the item "device ID" is associated with the item "status". In the present embodiment, information including a value of the item "medical institution ID", a value of the item "device ID", a value of the item "status", and a value of the item "expiration date", is the state management information.

The value of the item "device ID" is the identification information for identifying the electrocardiograph 10 dispatched to the medical institution identified by the medical institution ID. The value of the item "status" indicates the state of the dispatched electrocardiograph 10. More particularly, the value of the item "status" indicates whether or not the electrocardiograph 10 has been collected at the collection center 3. That is, in the state management database 220, the unique ID of the electrocardiograph 10 is associated with the collection status of the electrocardiograph 10 from the patient. The value of the item "expiration date" indicates the expiration date of the electrocardiograph 10 identified by the device ID.

In the example illustrated in FIG. 5, it may be seen that the electrocardiographs 10 respectively identified by the device ID "101" through the device ID "105" are dispatched to the medical institution identified by the medical institution ID "A01", and none of the electrocardiographs 10 has been collected at the collection center 3. In other words, the electrocardiographs 10 respectively identified by the device ID "101" through the device ID "105" any one of a state in which the electrocardiographs 10 are either stored in the medical institution at the dispatch destination, or in use by the patients who visited the medical institution at the dispatch destination.

In addition, it may be seen that the expiration dates of the electrocardiographs 10 identified by the device IDs "101" and "102", among the electrocardiographs 10 identified by the device ID "101" through the device ID "105", are March 10, 2021.

In the present embodiment, because the device ID and the expiration date are associated with each other as described above, the inventory management device 200 can send a warning notification to the medical institution to prohibit the use of the electrocardiographs 10 having expiration dates that have expired.

In the present embodiment, the medical institution at the dispatch destination of the electrocardiograph 10, the device ID of the electrocardiograph 10, and the status indicating the collection status of the electrocardiograph 10, are associated with each other in the state management database 220, but the association is not limited thereto. The association may be achieved using a plurality of types of databases. In other words, the state management database 220 may be implemented by a plurality of databases.

More particularly, the state management database 220 may be implemented by a dispatch destination database that stores the medical institution at the dispatch destination of the electrocardiograph 10 and the device ID of the electrocardiograph 10 in association with each other, and a collection status database that stores the device ID of the electrocardiograph 10 and the collection status of the electrocardiograph 10 in association with each other, for example.

In addition, the state management information stored in the state management database 220 may include a value indicating the current inventory quantity of the medical institution.

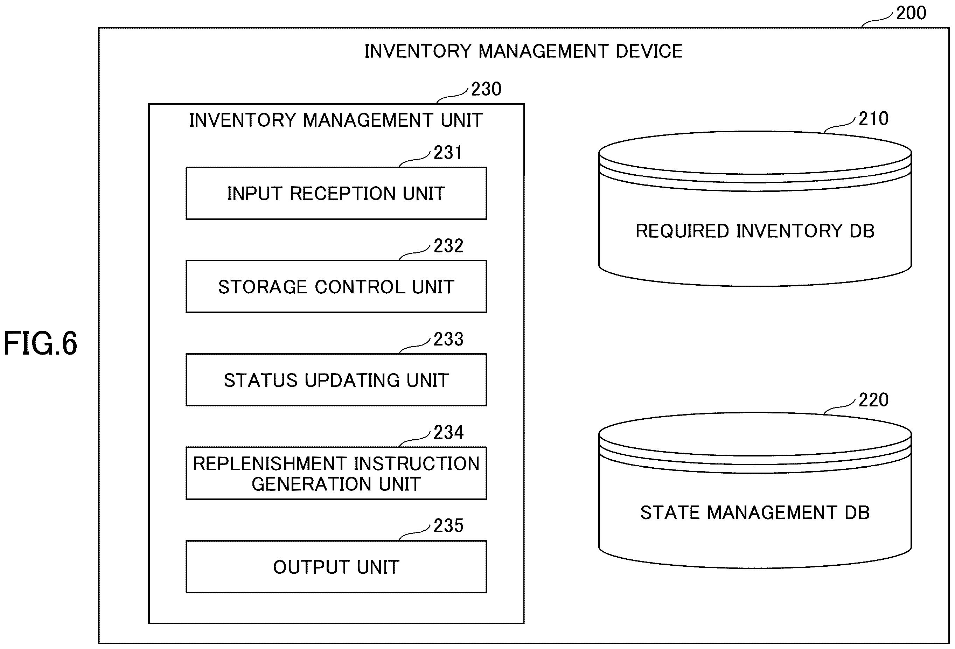

Next, the functions of the inventory management device 200 according to the present embodiment will be described. FIG. 6 is a diagram for explaining the functions of the inventory management device according to the first embodiment.

The inventory management device 200 according to the present embodiment includes an inventory management unit 230. The inventory management unit 230 includes an input reception unit 231, a storage control unit 232, a status updating unit 233, a replenishment instruction generation unit 234, and an output unit 235.

The input reception unit 231 receives an input with respect to the inventory management device 200. More particularly, the input reception unit 231 receives an input of the state information from the terminal device 300, and an input of the status of the electrocardiograph 10 or the like from the terminal device 400.

The storage control unit 232 stores the input received by the input reception unit 231 in the state management database 220.

The status updating unit 233 updates the value of the item "status" in the state management database 220 according to the input from the terminal device 400.

The replenishment instruction generation unit 234 refers to the required inventory database 210 and the state management database 220, and generates a replenishment instruction to replenish the inventory for each medical institution. More particularly, the replenishment instruction generation unit 234 refers to the required inventory database 210 and the state management database 220, and computes a replenishment quantity for setting the current inventory quantity of the medical institution to the required inventory quantity.

When the replenishment instruction generation unit 234 generates the replenishment instruction to replenish the inventory, the inventory management unit 230 may generate commercial transaction information for issuing a statement of delivery with respect to the medical institution, based on the replenishment instruction to replenish the inventory.

In addition, the replenishment instruction generation unit 234 according to the present embodiment may function as an inventory status generation unit. More particularly, the replenishment instruction generation unit 234 may generate information indicating the inventory status including both or one of the current inventory quantity of the electrocardiograph 10 and the inventory replenishment instruction based on the required inventory quantity included in the state management information stored in the state management database 220.

The output unit 235 outputs information to be displayed, with respect to the terminal device 300 and the terminal device 400. More particularly, the output unit 235 outputs the replenishment quantity computed by the replenishment instruction generation unit 234 to the terminal device 300 of the manufacturer and/or distributor 1. In addition, the output unit 235 may issue a statement of delivery based on the commercial transaction information generated according to the inventory replenishment instruction, an invoice in the case of the deposit inventory, or the like, with respect to the medical institution at the inventory replenishment destination, at a timing when the replenishment quantity is output to the terminal device 400.

Moreover, in the case where the replenishment instruction generation unit 234 generates the information indicating the inventory status including the replenishment instruction, the output unit 235 outputs the information indicating the inventory status to the terminal device 300.

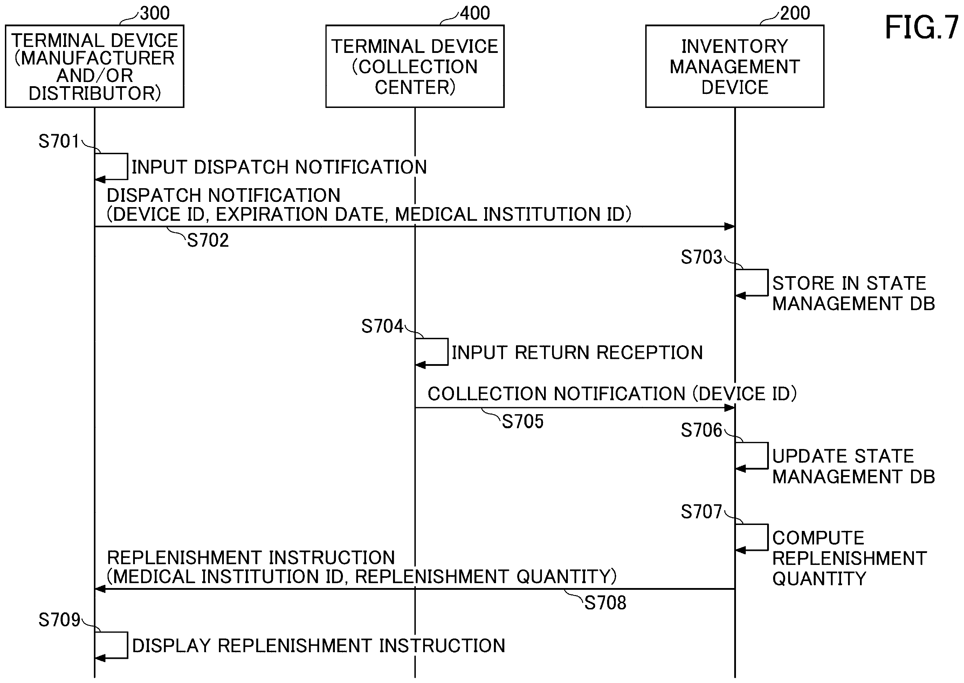

Next, an operation of the inventory management system 100 according to the present embodiment will be described with reference to FIG. 7. FIG. 7 is a sequence diagram for explaining the operation of the inventory management system according to the first embodiment.

In the inventory management system 100 according to the present embodiment, the terminal device 300 of the manufacturer and/or distributor 1 displays an input screen for transmitting a dispatch notification of the electrocardiograph 10 to the inventory management device 200 (step S701), and transmits the dispatch notification including the input information to the inventory management device 200 (step S702).

More particularly, the dispatch notification includes the device ID and the expiration date of the electrocardiograph 10 to be dispatched, and the medical institution ID of the medical institution at the dispatch destination of the electrocardiograph 10.

When the input reception unit 231 of the inventory management device 200 receives the dispatch notification, the storage control unit 232 stores the device ID, the expiration date associated with the device ID, and the status in the state management database 220 corresponding to the medical institution ID included in the dispatch notification (step S703). In this state, the status corresponding to the device ID is "uncollected".

Next, in the inventory management system 100, when the collection center 3 receives the return of the electrocardiograph 10 from a patient P, an input screen for transmitting a collection notification indicating the receipt of the returned electrocardiograph 10 to the inventory management device 200 is displayed on the terminal device 400 (step S704).

Then, the terminal device 400 transmits the collection notification, including the information input on the input screen, to the inventory management device 200 (step S705). More particularly, the collection notification includes the device ID read from the returned electrocardiograph 10.

When the inventory management device 200 receives the collection notification from the terminal device 400, the status updating unit 233 updates the value of the status in the state management database 220 (step S706).

More particularly, the status updating unit 233 identifies the state management database 220 of the medical institution including the device ID included in the collection notification, from among the state management databases 220 of the respective medical institutions. Then, the status updating unit 233 updates the status corresponding to the device ID included in the collection notification to "collected" in the identified state management database 220.

Next, the inventory management device 200 causes the replenishment instruction generation unit 234 to refer to the required inventory database 210, to compute the replenishment quantity of the electrocardiograph 10 with respect to the medical institution, from the required inventory quantity of the medical institution corresponding to the device ID included in the collection notification, and the quantity of collected electrocardiographs 10 (step S707).

Next, the inventory management device 200 causes the output unit 235 to output the replenishment instruction including the medical institution requiring replenishment of the electrocardiograph 10 and the replenishment quantity of the electrocardiograph 10, with respect to the terminal device 300 of the manufacturer and/or distributor 1 (step S708). More particularly, the replenishment instruction includes the medical institution ID of the medical institution at the replenishment destination of the electrocardiograph 10, and the replenishment quantity of the electrocardiograph 10.

When the terminal device 300 of the manufacturer and/or distributor 1 receives the replenishment instruction from the inventory management device 200, the terminal device 300 displays the replenishment instruction (step S709).

In the example illustrated in FIG. 7, the input of the dispatch notification and the display of the replenishment instruction are performed at the terminal device 300, but the input of the dispatch notification and the display of the replenishment instruction are not limited thereto. The input of the dispatch notification may be performed by the operation device 28 of the inventory management device 200 and input to the inventory management device 200. In addition, the replenishment instruction may be displayed on the display device 29 of the inventory management device 200.

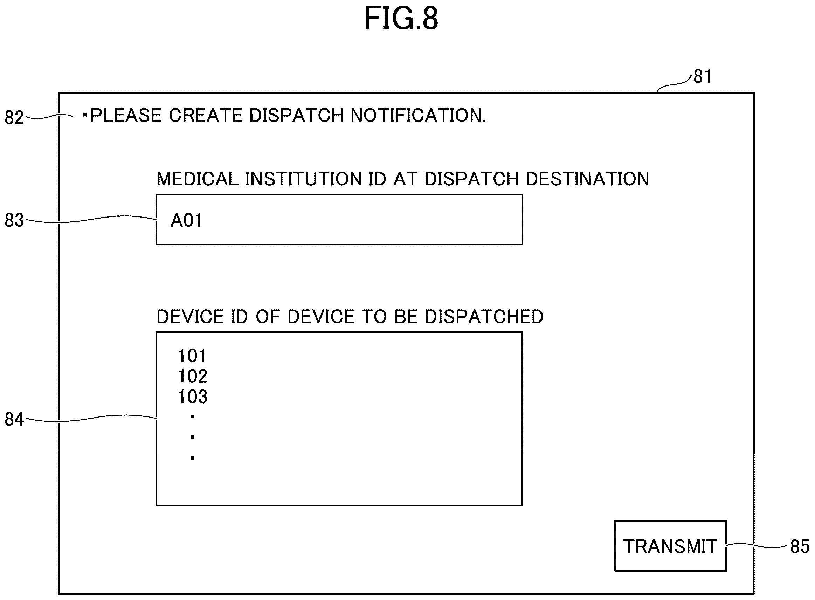

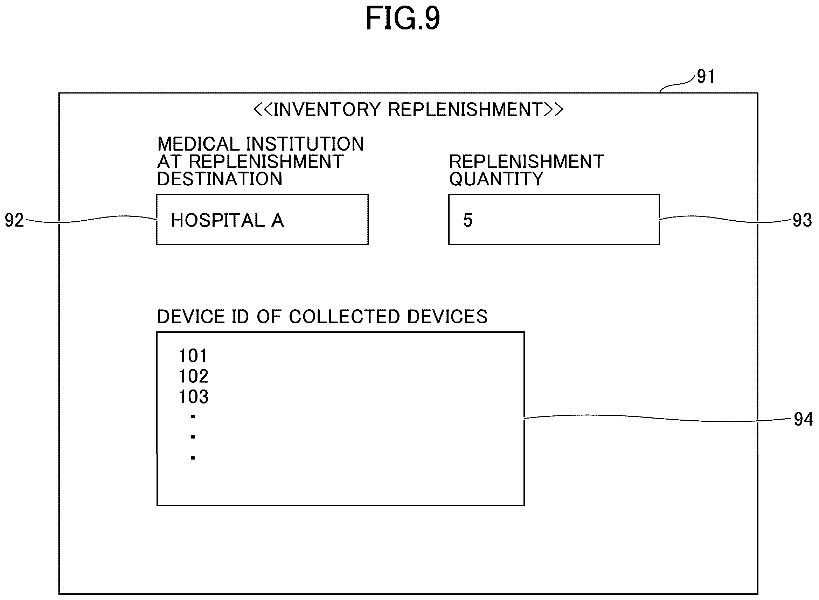

Hereinafter, display examples of the terminal device 300 according to the present embodiment will be described with reference to FIG. 8 and FIG. 9. The terminal device 300 according to the present embodiment may display a screen which will be described later, by a web browser or the like, for example. Further, an application distributed for using a service provided by the inventory management system 100 may be installed in the terminal device 300, and the screen which will be described later may be displayed by the application.

FIG. 8 is a diagram illustrating an example of an input screen for transmitting the dispatch notification according to the first embodiment. A screen 81 illustrated in FIG. 8 is an example of the input screen displayed on the terminal device 300 in step S701 of FIG. 7, for example.

The screen 81 includes a display field 82, input fields 83 and 84, and an operation button 85. In the display field 82, a message prompting creation of a dispatch notification is displayed.

The medical institution ID of the medical institution at the dispatch destination of the electrocardiograph 10 is input to the input field 83. The device ID and the expiration date of each electrocardiograph 10 dispatched to the medical institution identified by the medical institution ID input to the input field 83, are input to the input field 83.

In the example illustrated in FIG. 8, it may be seen that the electrocardiographs 10 identified by the device IDs "101" through "105" are dispatched to the medical institution identified by the medical institution ID "A01".

When the operation button 85 is manipulated after the medical institution ID and the device ID are input to the input fields 83 and 84 on the screen 81, a dispatch notification, including the medical institution ID "A01" and the device IDs "101" through "105", is created and transmitted to the inventory management device 200.

In the present embodiment, the device ID is input to the screen 81, but a method of obtaining the device ID is not limited thereto. The device ID may be acquired by reading a bar code attached to the electrocardiograph 10 or a packing sheet packing each electrocardiograph 10, for example.

FIG. 9 is a diagram illustrating a display example of the replenishment instruction according to the first embodiment. A screen 91 illustrated in FIG. 9 is an example of the screen displayed on the terminal device 300 in step S709 of FIG. 7, for example.

The screen 91 includes display fields 92, 93, and 94. The display field 92 displays the name of a medical institution at the replenishment destination (dispatch destination) of the electrocardiograph 10. The display field 93 displays the replenishment quantity of the electrocardiograph 10. The display field 94 displays the device ID of the electrocardiograph 10 collected from the medical institution displayed in the display field 92.

In the present embodiment, the device ID or the like of the electrocardiograph 10 scheduled to be dispatched to the medical institution may be displayed on the screen 91, for example.

As described above, according to the present embodiment, the medical institution uses the electrocardiograph 10 on the patient, and the patient returns the used electrocardiograph 10 to the collection center 3, so that the quantity of the electrocardiographs 10 lacking with respect to the required inventory quantity is automatically replenished from the manufacturer and/or distributor 1. Hence, according to the present embodiment, there is no need to perform the inventory management of the electrocardiograph 10 in the medical institution, and the load of the inventory management can be reduced.

Further, in the present embodiment, by associating the device ID of the electrocardiograph 10 with the medical institution ID at the dispatch destination when dispatching the electrocardiograph 10, the medical institution that used the electrocardiograph 10 and the used quantity of the electrocardiograph 10 can be notified to the manufacturer and/or distributor 1 when the electrocardiograph 10 is collected at the collection center 3. Thus, the manufacturer and/or distributor 1 does not need to check a use status, the collection status, or the like of the electrocardiograph 10 with respect to the medical institution 2 and the collection center 3, thereby reducing the load of the inventory management.

### (Second Embodiment)

Hereinafter, a second embodiment will be described with reference to the drawings. The second embodiment differs from the first embodiment in that the device ID, and an analysis result of analysis target data acquired from the electrocardiograph 10 identified by the device ID, are provided to the medical institution 2. In the following description of the second embodiment, differences from the first embodiment will be described, and constituent elements having the same functional configurations as those of the first embodiment will be designated by the same reference numerals as the first embodiment, and a description thereof will be omitted.

The analysis target data according to the present embodiment includes the electrocardiogram waveform data read from the electrocardiograph 10, data obtained by processing the electrocardiogram waveform data to suit input to analysis software, or the like.

In addition, analysis result data, that is an analysis result of the analysis target data by the analysis software, includes an analysis result, presence or absence of an error, error information, or the like. The error information in the present embodiment may include both information on the error of the electrocardiograph 10 itself and an abnormality generated at the time of measurement, for example, and may further include an abnormal value of the electrocardiogram waveform data related to a diagnosis.

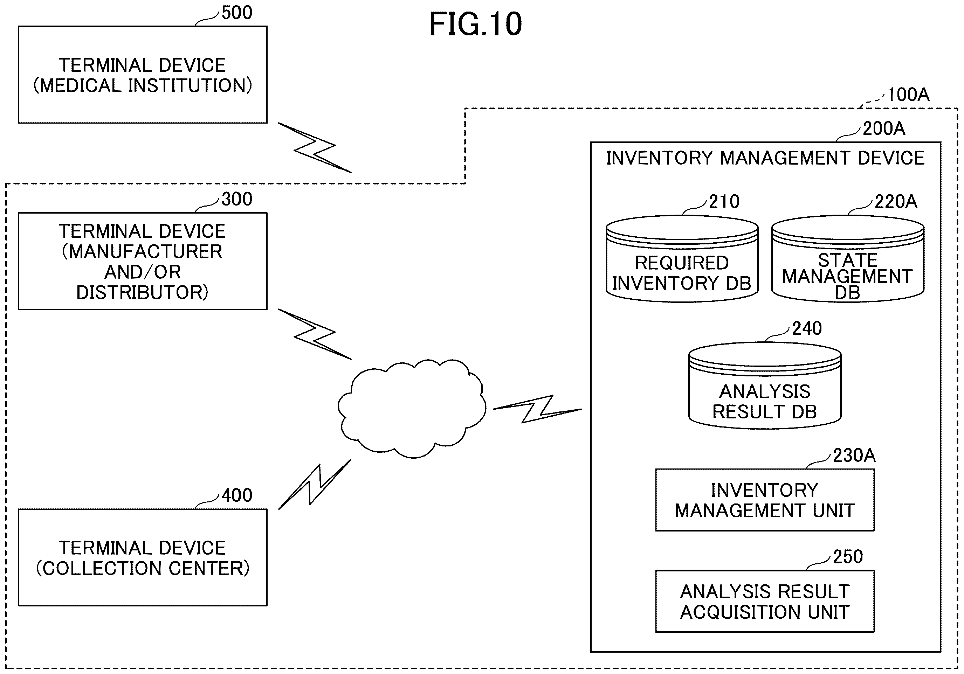

FIG. 10 is a diagram illustrating an example of a system configuration of the inventory management system according to the second embodiment. An inventory management system 100A according to the present embodiment includes an inventory management device 200A, and terminal devices 300 and 400. Moreover, in the inventory management system 100A according to the present embodiment, the inventory management device 200A communicates with a terminal device 500. The terminal device 500 is mainly used by the medical institution that employs the inventory management system 100A.

The inventory management device 200A according to the present embodiment includes a required inventory database 210, a state management database 220A, an analysis result database 240, an inventory management unit 230A, and an analysis result acquisition unit 250.

The state management database 220A stores state management information indicating the state of the electrocardiograph 10, including the use status of the electrocardiograph 10 by the patient.

The analysis result database 240 stores analysis result data, that is an analysis result of the analysis target data acquired from the storage device of the electrocardiograph 10 collected at the collection center 3, and activity record information 11 indicating an activity record during measurement of the electrocardiogram waveform data provided from the patient, in association with each other.

In the present embodiment, the analysis of the analysis target data may be performed by an analyzer or the like provided externally with respect to the inventory management system 100.

The analysis result acquisition unit 250 acquires the analysis result data from the external analyzer or the like, and stores the acquired data in the analysis result database 240. In this state, the analysis result data may include the device ID of the electrocardiograph 10 from which the analysis target data is read.

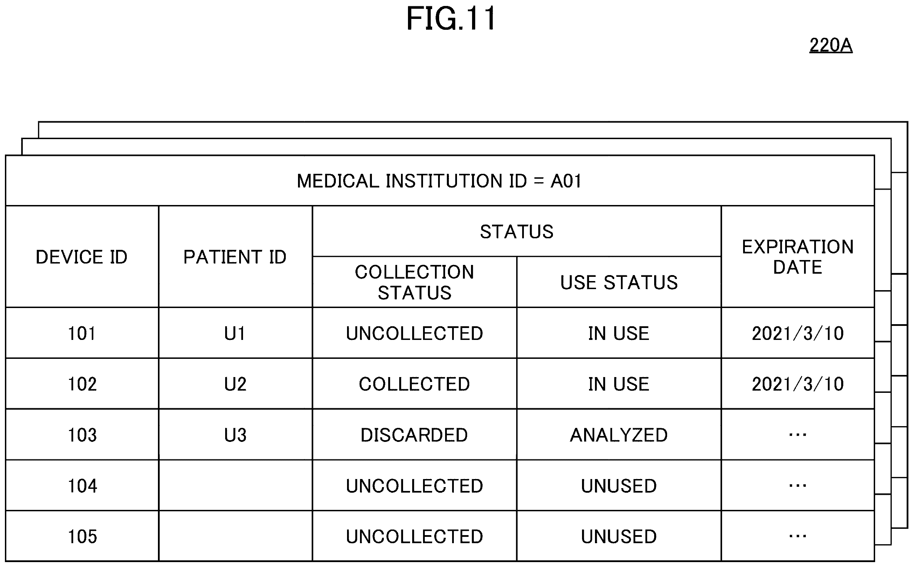

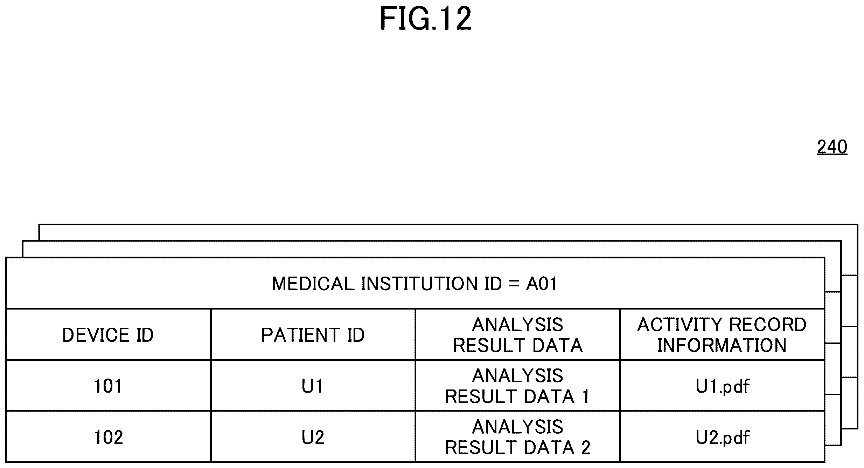

Hereinafter, the state management database 220A and the analysis result database 240 according to the present embodiment will be described with reference to FIG. 11 and FIG. 12.

FIG. 11 is a diagram illustrating an example of the state management database according to the second embodiment. The state management database 220A according to the present embodiment includes, as information items, a medical institution ID, a device ID, a status, a patient ID, and a status, and the item "status" includes the items "collection status", "use status", and "expiration date".

The value of the item "patient ID" is identification information for identifying the patient, and in the example illustrated in FIG. 11, indicates the identification information of the patient who uses the electrocardiograph 10 identified by the device ID.

The value of the item "collection status" included in the item "status" indicates the collection status of the electrocardiograph 10 at the collection center 3. The value of the item "use status" indicates the use status of the electrocardiograph 10 by the patient.

In the example illustrated in FIG. 11, the electrocardiograph 10 identified by the device ID "101" is in a state used by the patient identified by the patient ID "U1", and is not collected at the collection center 3.

In addition, the electrocardiograph 10 identified by the device ID "102" is collected at the collection center 3 after being used by the patient identified by the patient ID "U2", and the electrocardiogram waveform data or the like read from the electrocardiograph 10 is being analyzed.

Moreover, the electrocardiograph 10 identified by the device ID "103" is collected at the collection center 3 after being used by the patient identified by the patient ID "U3", and is discarded after the analysis of the electrocardiogram waveform data or the like read from the electrocardiograph 10 is completed.

Further, it may be seen that the electrocardiographs 10 identified by the device IDs "104" and "105" are unused, and are stored in the medical institution identified by the medical institution ID "A01".

FIG. 12 is a diagram illustrating an example of the analysis result database according to the second embodiment. The analysis result database 240 according to the present embodiment may be provided for each medical institution. The analysis result database 240 includes, as information items, a medical institution ID, a device ID, a patient ID, analysis result data, and activity record information, and the item "device ID" and each of the other items are associated with each other.

The value of the item "analysis result data" is analysis result data indicating the analysis result of the analysis target data read from the storage device of the electrocardiograph 10 identified by the device ID.

The value of the item "behavior record information" is information indicating an activity record of the patient identified by the patient ID during measurement of the electrocardiogram waveform data.

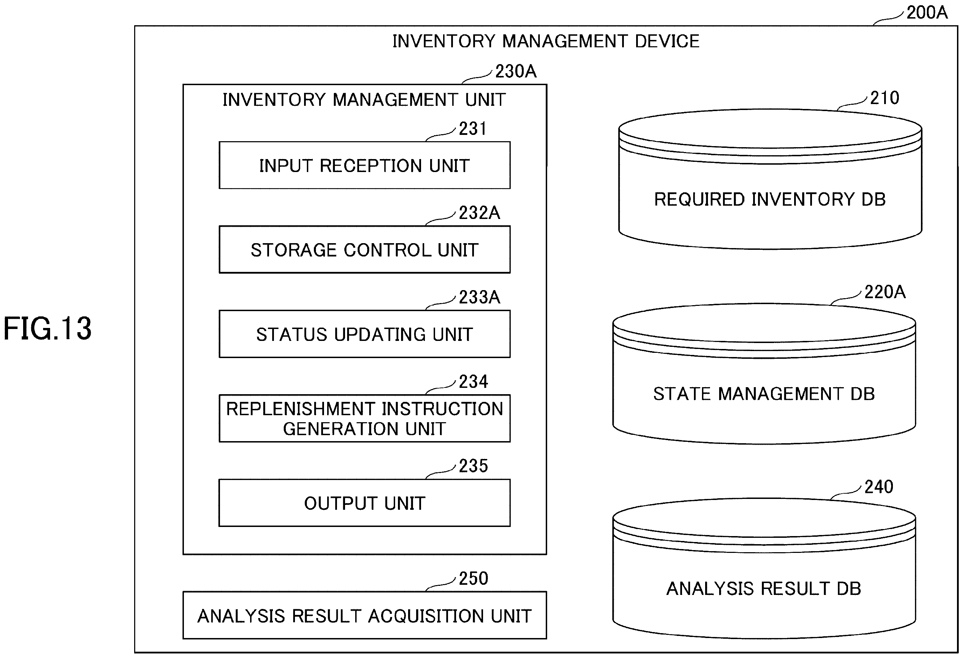

Next, functions of the inventory management device 200A according to the present embodiment will be described with reference to FIG. 13. FIG. 13 is a diagram for explaining the functions of the inventory management device according to the second embodiment.

The inventory management device 200A according to the present embodiment includes an inventory management unit 230A, and an analysis result acquisition unit 250. The inventory management unit 230A includes an input reception unit 231, a storage control unit 232A, a status updating unit 233A, a replenishment instruction generation unit 234, and an output unit 235.

The storage control unit 232A stores information input from the terminal device 500 in the state management database 220. In addition, the storage control unit 232A stores the analysis result data acquired from the external analyzer in the analysis result database 240.

The status updating unit 233A updates the state management database 220A according to information input from the terminal device 500 and the processing performed by the analysis result acquisition unit 250.

The analysis result acquisition unit 250 acquires the analysis result data of the analysis target data analyzed by the external analyzer.

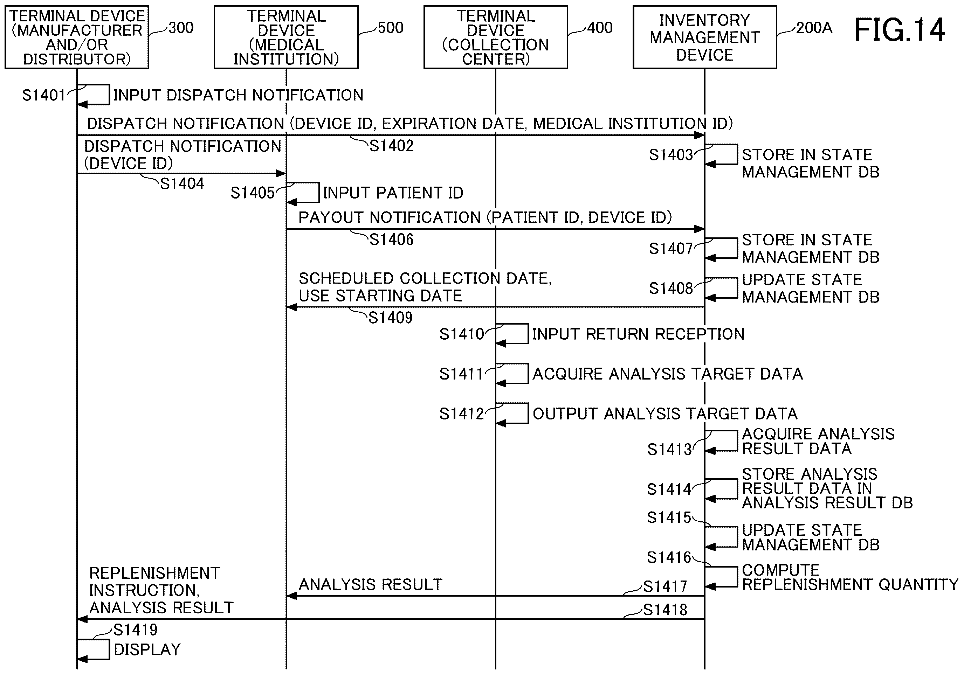

Next, ab operation of the inventory management system 100A according to the present embodiment will be described with reference to FIG. 14. FIG. 14 is a sequence diagram illustrating the operation of the inventory management system according to the second embodiment.

Because processes of step S1401 through step S1403 in FIG. 14 are the same as the processes from step S701 through step S703 in FIG. 7, a description thereof will be omitted.

Next, the terminal device 300 transmits a notice of delivery of the electrocardiograph 10 to the terminal device 500 of the medical institution at the dispatch destination of the electrocardiograph 10 (step S1404). The delivery notification includes the device ID. The delivery notification may include the medical institution ID together with the device ID.

When the patient uses the electrocardiograph 10 delivered to the medical institution, a person in charge at the medical institution inputs, from the terminal device 500, the patient ID on the input screen for associating the patient ID with the device ID (step S1405) .

Next, the terminal device 500 transmits a payout notification in which the patient ID and the device ID are associated with each other, to the inventory management device 200A (step S1406).

When the inventory management device 200A receives the payout notification, the storage control unit 232A identifies the state management database 220A including the device ID included in the payout notification, and stores the patient ID in association with the device ID in the identified state management database 220A (step S1407).

Next, the inventory management device 200A causes the status updating unit 233A to update the value of the item "collection status" to "uncollected" (refer to FIG. 11), and to update the value of the item "use status" to "in use", among the items "status" in the state management database 220A (step S1408).

Next, the inventory management device 200A notifies a scheduled return date (scheduled collection date) of the electrocardiograph 10 used by the patient, and a date on which the payout notification is received, to the terminal device 500 (step S1409). In other words, the date on which the payout notification is received is the date (use start date) on which the patient starts to use the delivered electrocardiograph 10. In the present embodiment, both the scheduled collection date and the use start date may be notified to the medical institution, or one of these dates may be notified to the medical institution. In addition, in the present embodiment, the use start date may be notified to the medical institution only when the scheduled collection date is not set.

The scheduled return date may be determined in advance when the patient is to use the electrocardiograph 10. Moreover, when the return notification is not received from the terminal device 400 of the collection center 3 even after the scheduled return date, for example, the inventory management device 200A may output an alarm indicating that the electrocardiograph 10 has not been returned, with respect to the medical institution that paid out the electrocardiograph 10 to the patient. Further, in a case where the scheduled collection date is not set, the inventory management device 200A may output an alarm with respect to the medical institution, using the use start date as a reference.

Next, when the electrocardiograph 10 returned from the patient identified by the patient ID is received, the terminal device 400 of the collection center 3 displays an input screen for transmitting the collection notification to the inventory management device 200, and the device ID of the collected electrocardiograph 10 and the patient ID are input to the terminal device 400 (step S1410).

Next, in the collection center 3, the electrocardiogram waveform data is read from the storage device of the returned electrocardiograph 10, and the activity record information 11 is provided from the patient. The terminal device 400 acquires the analysis target data including such information (step S1411), and outputs the analysis target data to the external analyzer (step S1412). The device ID of the electrocardiograph 10 from which the electrocardiogram waveform data is read, and the patient ID of the patient who used the electrocardiograph 10, are included in the analysis target data acquired in step S1410.

When the inventory management device 200A acquires (receives) the analysis result data from the analyzer to which the terminal device 400 transmitted the analysis target data (step S1413), the inventory management device 200A causes the storage control unit 232A to store the analysis result data in the analysis result database 240 (step S1414). More particularly, the storage control unit 232A stores the device ID included in the analysis result data and the patient ID, the medical institution ID corresponding to the device ID and the patient ID, and the activity record information corresponding to the patient ID, in the analysis result database 240 in association with one another.

Next, the inventory management device 200A causes the status updating unit 233A to update the state management database 220A (step S1415) .

More particularly, the status updating unit 233A updates the value of the item "collection status" to "collected", and the value of the item "use status" to "analyzed", among the items "status" in the state management database 220A (refer to FIG. 11).

In a case where the electrocardiograph 10 is discarded after the analysis of the analysis target data, the inventory management device 200A may receive a discard notification from the terminal device 400, and set the value of the item "collection status" to "discarded".

Next, the inventory management device 200A causes the replenishment instruction generation unit 234 to refer to the required inventory database 210, and to compute the replenishment quantity of the electrocardiograph 10 with respect to the medical institution, from the required inventory quantity of the medical institution corresponding to the device ID included in the collection notification, and the quantity of the collected electrocardiographs 10 (step S1416) .

Next, the inventory management device 200A causes the output unit 235 to transmit the analysis result acquired by the analysis result acquisition unit 250 to the terminal device 500 of the medical institution (step S1417).

In addition, the inventory management device 200A causes the output unit 235 to transmit the replenishment instruction and the analysis result to the terminal device 300 (step S1418).

When the replenishment instruction is received, the terminal device 300 displays the replenishment instruction together with the analysis result (step S1419) .

In the present embodiment, the analysis target data is analyzed by the external analyzer, but the analysis is not limited thereto. The analyzer may be included in the inventory management system 100A. Moreover, the analyzer that analyzes the analysis target data may be provided in the inventory management device 200A.

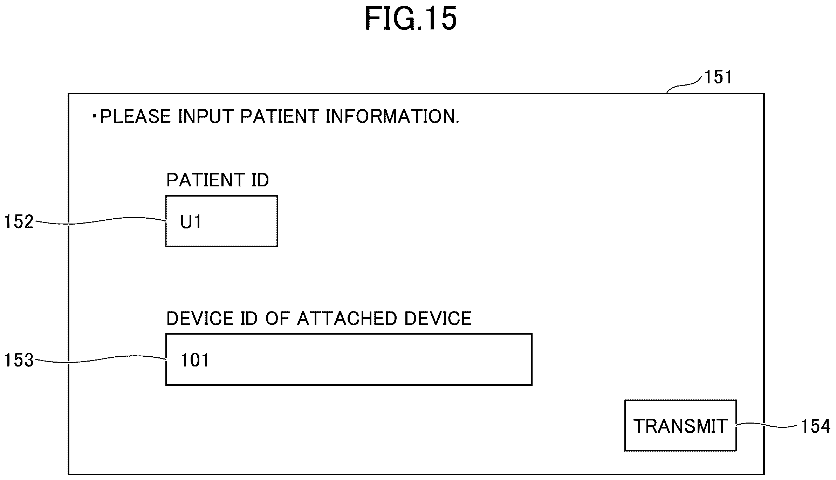

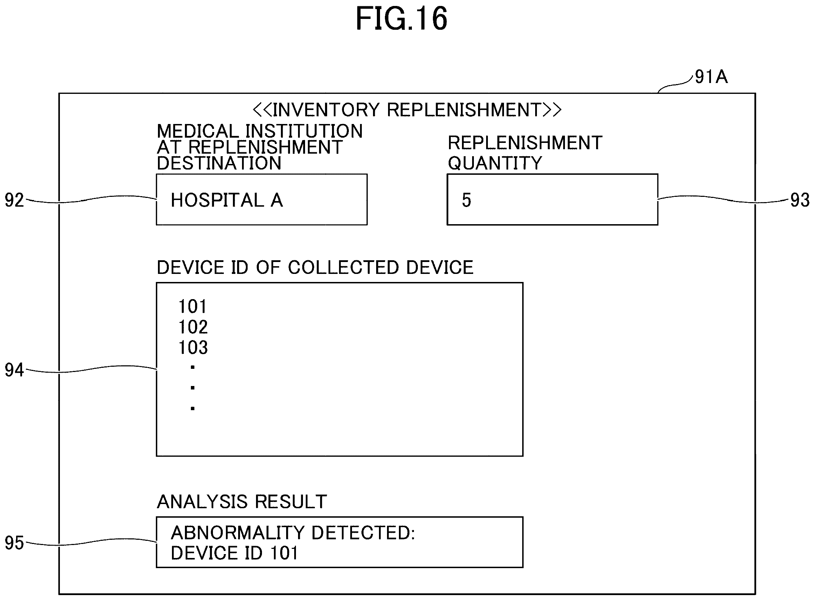

Hereinafter, a display example according to the present embodiment will be described with reference to FIG. 15 and FIG. 16. FIG. 15 is a diagram illustrating an example of the input screen for transmitting the payout notification according to the second embodiment.

A screen 151 illustrated in FIG. 15 is displayed on the terminal device 500 in step S1405 of FIG. 14, for example.

The screen 151 includes input fields 152 and 153, and an operation button 154. The patient ID of the patient who uses the electrocardiograph 10 is input to the input field 152. The device ID of the electrocardiograph 10 to be used is input to the input field 153.

When the operation button 154 is manipulated after the inputs with respect to the input fields 152 and 153 are made, the terminal device 500 transmits the payout notification to the inventory management device 200A.

In the screen 151, only the patient ID is input as the information for identifying the patient, but the input information is not limited thereto. Information, such as gender, age, past medical history, or the like of the patient, may be input on the screen 151, and included in the analysis target information.

FIG. 16 is a diagram illustrating a display example of the replenishment instruction according to the second embodiment. A screen 91A illustrated in FIG. 16 is an example of the screen displayed on the terminal device 300 in step S1417 of FIG. 14.

The screen 91A includes display fields 92, 93, 94, and 95. The analysis result acquired by the analysis result acquisition unit 250 is displayed in the display field 95. In the example illustrated in FIG. 16, the device ID of the electrocardiograph 10 determined to include an error as a result of the analysis, is displayed.

In the present embodiment, by notifying the analysis result of the electrocardiogram waveform data acquired from the electrocardiograph 10 to a person in charge at the manufacturer and/or distributor 1 as described above, it is possible to prompt the medical institution to replenish the patient who uses the electrocardiograph 10 in which the error occurred, with a new electrocardiograph 10, for example.

Further, in the present embodiment, it is possible to track a distribution path from the dispatch to the return of the electrocardiograph 10 in which the error occurred.

In the embodiment described above, the electrocardiograph 10 is described as an example of the medical device that is a target of inventory management by the inventory management system, however, medical device that is the target of the inventory management by the inventory management system according to the present embodiment is not limited to the electrocardiograph 10.

The inventory management system described above can be applied to any medical device, as long as the medical device is a single-use medical device that is collected and discarded after being used by the patient. Other examples of the single-use medical device that is collected and discarded after being used by the patient include a blood glucose sensor chip for blood glucose monitoring having a measurement result notification function, an electrode material, or the like, for example.

The present invention is not limited to the configurations of the embodiments described above, and combinations with other elements or the like may be made. The present invention may be modified without departing from the scope of the present invention, and can be suitably determined to suit the form of the application.

Further, the present international application is based on and claims priority to Japanese Patent Application No. 2021-043822, filed on March 17, 2021, and the entire contents of which are incorporated herein by reference.

### DESCRIPTION OF REFERENCE NUMERALS

- 100, 100A: inventory management system
- 200, 200A: inventory management device
- 210: required inventory database
- 220, 220A: state management database
- 230: inventory management unit
- 231: input reception unit
- 232: storage control unit
- 233: status updating unit
- 234: replenishment instruction generation unit
- 235: output unit
- 240: analysis result database
- 250: analysis result acquisition unit
- 300, 400, 500: terminal device

## Claims

1. An inventory management system comprising an inventory management device and a terminal device, wherein the inventory management device includes:
an inventory status generation unit configured to generate information indicating an inventory status of a medical device for each of a plurality of medical institutions, by referring to a required inventory storage unit storing information indicating a required inventory quantity of the medical device for each of the plurality of medical institutions, and a state management storage unit storing state management information associating a unique ID of the medical device dispatched to one medical institution with a collection status of the medical device paid out from the one medical institution to a patient for each of the plurality of medical institutions; and
an output unit configured to output the information indicating the inventory status to the terminal device.

2. The inventory management system as claimed in claim 1, wherein the information indicating the inventory status includes a current inventory quantity of the medical device and/or a replenishment instruction to replenish the inventory of the medical device.

3. The inventory management system as claimed in claim 2, comprising:
a storage control unit configured to receive an input of a patient ID for identifying the patient wearing the medical device and store the patient ID in the state management storage unit in association with the unique ID of the medical device,
wherein the output unit outputs a scheduled collection date of the medical device from the patient wearing the medical device and/or a use starting date of the medical device by the patient, to the terminal device.

4. The inventory management system as claimed in claim 2 or 3, comprising:
an analysis result acquisition unit configured to acquire analysis result data indicating an analysis result of analysis target data measured by the medical device dispatched to the one medical institution for each of the plurality of medical institutions,
wherein the output unit outputs the analysis result data acquired by the analysis result acquisition unit to the terminal device together with the replenishment instruction.

5. The inventory management system as claimed in claim 4, wherein the analysis result data includes information related to an error of the medical device.

6. The inventory management system as claimed in any one of claims 1 to 5, wherein the medical device is a single-use medical device.

7. The inventory management system as claimed in any one of claims 1 to 6, wherein the medical device is an electrocardiograph.

8. The inventory management system as claimed in claim 7, wherein the analysis target data measured by the medical device includes electrocardiogram waveform data measured by the electrocardiograph.

9. An inventory management device comprising:
an inventory status generation unit configured to generate information indicating an inventory status of a medical device for each of a plurality of medical institutions, by referring to a required inventory storage unit storing information indicating a required inventory quantity of the medical device for each of the plurality of medical institutions, and a state management storage unit storing state management information associating a unique ID of the medical device dispatched to one medical institution with a collection status of the medical device paid out from the one medical institution to a patient for each of the plurality of medical institutions; and
an output unit configured to output the information indicating the inventory status.

10. An inventory management method implemented by an inventory management system including an inventory management device and a terminal device, the inventory management method comprising:
generating, by the inventory management device, information indicating an inventory status of a medical device for each of a plurality of medical institutions, by referring to a required inventory storage unit storing information indicating a required inventory quantity of the medical device for each of the plurality of medical institutions, and a state management storage unit storing state management information associating a unique ID of the medical device dispatched to one medical institution with a collection status of the medical device paid out from the one medical institution to a patient for each of the plurality of medical institutions; and
outputting, by the inventory management device, the information indicating the inventory status to the terminal device.

11. An inventory management program for causing a computer to perform a process comprising:
generating information indicating an inventory status of a medical device for each of a plurality of medical institutions, by referring to a required inventory storage unit storing information indicating a required inventory quantity of the medical device for each of the plurality of medical institutions, and a state management storage unit storing state management information associating a unique ID of the medical device dispatched to one medical institution with a collection status of the medical device paid out from the one medical institution to a patient for each of the plurality of medical institutions; and
outputting the information indicating the inventory status.
